(19) Europäisches Patentamt / European Patent Office / Office européen des brevets

(11) **EP 3 727 628 B1**

(12) **FASCICULE DE BREVET EUROPEEN**

(45) Date de publication et mention
de la délivrance du brevet:
**03.11.2021 Bulletin 2021/44**

(21) Numéro de dépôt: **18833103.7**

(22) Date de dépôt: **17.12.2018**

(51) Int Cl.:
**B01J 20/18** (2006.01)      **B01J 20/30** (2006.01)
**B01D 15/08** (2006.01)      **B01D 15/18** (2006.01)
**C07C 7/13** (2006.01)

(86) Numéro de dépôt international:
**PCT/FR2018/053328**

(87) Numéro de publication internationale:
**WO 2019/122650 (27.06.2019 Gazette 2019/26)**

(54) **ADSORBANTS ZÉOLITHIQUES À BASE DE BARYUM, STRONTIUM, POTASSIUM ET SODIUM, LEUR PROCÉDÉ DE PRÉPARATION ET LEURS UTILISATIONS**

ZEOLITHISCHE ADSORPTIONSMITTEL ENTHALTEND BARYUM, STRONTIUM, POTASSIUM AND SODIUM, VERFAHREN ZUR HERSTELLUNG DAVON UND VERWENDUNG DAVON

ZEOLITIC ADSORBENTS CONTAINING BARYUM, STRONTIUM, POTASSIUM AND SODIUM, METHOD FOR PREPARATION THEREOF AND USES OF THE SAME

(84) Etats contractants désignés:
**AL AT BE BG CH CY CZ DE DK EE ES FI FR GB GR HR HU IE IS IT LI LT LU LV MC MK MT NL NO PL PT RO RS SE SI SK SM TR**

(30) Priorité: **22.12.2017 FR 1763033**

(43) Date de publication de la demande:
**28.10.2020 Bulletin 2020/44**

(73) Titulaires:
• **ARKEMA FRANCE**
  **92700 Colombes (FR)**
• **IFP Energies nouvelles**
  **92500 Rueil-Malmaison (FR)**

(72) Inventeurs:
• **LAROCHE, Catherine**
  **92852 Rueil-Malmaison Cedex (FR)**
• **BOUVIER, Ludivine**
  **64300 Orthez (FR)**
• **PEREZ-PELLITERO, Javier**
  **69007 Lyon (FR)**
• **LABEDE, Marie-Laurence**
  **64230 Lescar (FR)**

(74) Mandataire: **Bandpay & Greuter**
**30, rue Notre-Dame des Victoires**
**75002 Paris (FR)**

(56) Documents cités:
**WO-A1-2017/005907      FR-A1- 2 329 623**

**Description**

DOMAINE TECHNIQUE

L'invention concerne des adsorbants à base de cristaux agglomérés de zéolithe X comprenant du baryum, du strontium et du potassium, leur procédé de préparation et leurs utilisations.

Ces adsorbants peuvent être utilisés plus particulièrement pour la production en phase liquide ou phase gaz de *para*-xylène très pur à partir d'une charge d'hydrocarbures aromatiques contenant des isomères à 8 atomes de carbone.

TECHNIQUE ANTERIEURE

Il est connu dans l'art antérieur que des adsorbants comprenant des aluminosilicates cristallins peuvent être utilisés pour séparer certains hydrocarbures à partir de mélanges les contenant. Dans le domaine de la séparation d'hydrocarbures aromatiques et en particulier la séparation d'isomères en C8 aromatiques, il est généralement reconnu que l'utilisation de cations particuliers dans les sites cationiques d'aluminosilicates cristallins zéolithiques améliore la sélectivité de la zéolithe pour un des isomères en C8-aromatique. Cette adsorption différenciée au sein de la zéolithe permet la séparation des différents isomères en C8-aromatique, ce qui est utilisé industriellement pour la production de *para*-xylène très pur à partir d'une charge d'hydrocarbures aromatiques contenant des isomères à 8 atomes de carbone.

Ainsi, l'utilisation d'adsorbants zéolithiques constitués de zéolithes X ou Y comprenant, outre des cations sodium, des cations baryum, potassium ou strontium, seuls ou en mélanges, pour adsorber sélectivement en phase liquide le *para*-xylène dans un mélange d'hydrocarbures aromatiques, est bien connue de l'art antérieur.

Les brevets US 3 558 730, US 3 663 638 et US 3 960 774 montrent que des adsorbants zéolithiques comprenant des aluminosilicates de structure faujasite (FAU) à base de sodium et de baryum ou à base de sodium, de baryum et de potassium, sont efficaces pour la séparation du *para*-xylène présent dans des coupes aromatiques en C8 (coupes comprenant des hydrocarbures aromatiques à 8 atomes de carbone). Les adsorbants ci-dessus sont de préférence utilisés comme agents d'adsorption dans les procédés en phase liquide, notamment de type contre-courant simulé, similaires à ceux décrits dans le brevet US 2 985 589 et qui s'appliquent, entre autres, aux coupes aromatiques en C8.

Les brevets US 3 558 730, US 3 626 020 et US 3 997 620 montrent que des adsorbants zéolitiques comprenant des aluminosilicates de structure faujasite (FAU) à base de sodium et de baryum ou à base de sodium, de baryum et de strontium, sont efficaces pour la séparation du *para*-xylène présent dans des coupes aromatiques en C8 (coupes comprenant des hydrocarbures aromatiques à 8 atomes de carbone).

Le brevet WO 2017/005907 décrit un adsorbant zéolithique comprenant des cristaux de zéolithe X et comprenant du baryum, du potassium et du sodium, dans lequel le ratio molaire $K_2O$ / ($K_2O$ + BaO + $Na_2O$) est compris entre 8,0% et 8,6% et utilisé dans les procédés similaires de séparation des coupes aromatiques.

Cependant, de manière générale, les propriétés d'adsorption des zéolithes pour les hydrocarbures aromatiques à 8 atomes de carbone (xylènes et éthylbenzène) varient de manière très fine en fonction de la taille et de la forme des pores ainsi que de la position des cations à l'intérieur de la structure qui influent à la fois sur le champ électrostatique présent à l'intérieur de la zéolithe et sur la forme du volume accessible dans les pores. D'autres paramètres, tels que la polarisabilité des cations et des molécules ou la flexibilité de la structure peuvent également avoir une influence. Il est donc extrêmement difficile de prévoir théoriquement et avec précision les caractéristiques d'adsorption d'une zéolithe vis-à-vis des hydrocarbures aromatiques à 8 atomes de carbone.

Pour améliorer la sélectivité d'adsorption de zéolithes ayant la structure faujasite pour les isomères aromatiques en C8, de nombreuses études ont fait mention de l'influence du rapport Si/Al de la zéolithe, de la nature des cations d'échange, ainsi que de leur teneur en eau. De la même manière, il est très difficile de prédire le degré d'amélioration parce que ces facteurs exercent des actions combinées sur les caractéristiques d'adsorption des zéolithes. En particulier, il est difficile de prévoir l'impact de la proportion relative des cations choisis parmi sodium, baryum, strontium et potassium dans le cas d'une zéolithe de structure faujasite (FAU), et plus précisément dans le cas d'une zéolithe de structure faujasite (FAU) de type X.

Le brevet US 3 997 620 présente des agglomérés, dans lesquels le liant d'agglomération n'est pas zéolithisé, lesdits agglomérés étant échangés au baryum et au strontium, de sorte que le ratio pondéral Ba/Sr est compris entre 1:1 et 15:1. Les exemples de ce brevet montrent que la pureté de l'extrait est améliorée avec l'addition de cations strontium. Cependant les sélectivités PX/MX et PX/OX se voient fortement diminuées, ce qui est problématique pour la production de paraxylène de haute pureté.

La synthèse des zéolithes conduit à des cristaux (généralement sous forme de poudre) dont l'emploi à l'échelle industrielle est particulièrement malaisé (pertes de charges importantes lors des manipulations). On préfère alors les formes agglomérées de ces cristaux, sous forme de grains, de filés et autres agglomérés, ces dites formes pouvant être obtenues par extrusion, pastillage, et autres techniques d'agglomération connues de l'homme du métier. Ces agglomérés ne présentent pas les inconvénients inhérents aux matières pulvérulentes.

Ces agglomérés, qu'ils soient sous forme de plaquettes, de billes, d'extrudés, et autres, sont en général formés de cristaux de zéolithe(s), qui constituent l'élément actif (au sens de l'adsorption) et d'un liant destiné à assurer la cohésion des cristaux sous forme d'agglomérés et de leur conférer une résistance mécanique suffisante pour résister aux vibrations et aux mouvements auxquels ils sont soumis au cours des opérations de séparations des isomères des coupes aromatiques en C8. Cependant, les propriétés d'adsorption de ces agglomérés sont évidemment réduites par rapport à la poudre de cristaux, en raison de la présence de liant d'agglomération inerte vis-à-vis de l'adsorption. Divers moyens ont déjà été proposés pour pallier cet inconvénient du liant d'agglomération d'être inerte quant aux performances d'adsorption, parmi lesquels, la transformation de la totalité ou d'au moins une partie du liant d'agglomération en zéolithe active du point de vue de l'adsorption. Cette opération est maintenant bien connue de l'homme du métier, par exemple sous la dénomination de « zéolithisation ». Pour effectuer facilement cette opération, on utilise des liants zéolithisables, le plus souvent des argiles appartenant à la famille de la kaolinite, et de préférence préalablement calcinés à des températures généralement comprises entre 500°C et 700°C.

La demande de brevet FR 2 789 914 décrit par exemple un procédé de fabrication d'agglomérés de zéolithe X, de rapport Si/Al compris entre 1,15 et 1,5, contenant du baryum et éventuellement du potassium. Les agglomérés ainsi obtenus, après zéolithisation du liant, présentent, du point de vue de l'adsorption du *para*-xylène contenu dans les coupes aromatiques en C8, des propriétés améliorées par rapport à des adsorbants préparés à partir de la même quantité de zéolithe X et de liant, mais dont le liant n'est pas zéolithisé.

Les facteurs importants qui influencent les performances d'un procédé de séparation par adsorption englobent notamment la sélectivité d'adsorption, la capacité d'adsorption et la cinétique de transfert de matière qui définit les vitesses d'adsorption et de désorption des différents composés. L'adsorbant doit donc présenter de bonnes propriétés de transfert de matière afin de garantir un nombre de plateaux théoriques suffisants pour réaliser une séparation efficace des espèces en mélange, comme l'indique Ruthven dans l'ouvrage intitulé « Principles of Adsorption and Adsorption Processes » (« Principes de l'Adsorption et des Procédés d'Adsorption »), John Wiley & Sons, (1984), pages 326 et 407. Ruthven indique (*ibid.,* page 243), que, dans le cas d'un adsorbant aggloméré, le transfert de matière global dépend de l'addition de la résistance diffusionnelle intra-cristalline et de la résistance diffusionnelle entre les cristaux. La résistance diffusionnelle intra-cristalline est proportionnelle au carré des rayons des cristaux et inversement proportionnelle à la diffusivité des molécules intracristalline.

La résistance diffusionnelle entre les cristaux (également appelée résistance macroporeuse) est quant à elle proportionnelle au carré des rayons des agglomérés et inversement proportionnelle à la diffusivité des molécules dans les macropores. Pour une structure zéolithique donnée, une taille d'aggloméré donnée et une température de fonctionnement donnée, les diffusivités sont fixées, et le seul moyen d'améliorer le transfert de matière consiste à réduire le diamètre des cristaux. Un gain sur le transfert global sera ainsi obtenu en réduisant la taille des cristaux.

Par conséquent, l'homme du métier s'attend à ce que des adsorbants zéolithiques agglomérés présentant à la fois une bonne capacité d'adsorption des xylènes et une bonne sélectivité pour le *para*-xylène, possèdent de très bonnes propriétés de séparation des xylènes lorsqu'ils sont réalisés à partir de petits cristaux de zéolithe dans les procédés en phase liquide de séparation du *para*-xylène contenu dans les coupes aromatiques en C8, par exemple de type contre-courant simulé. L'homme du métier est cependant dans l'impossibilité de définir *a priori* ou théoriquement et avec précision les caractéristiques d'adsorption d'une zéolithe FAU, notamment de type X, comprenant du baryum et du potassium, et éventuellement d'autres cations tels que sodium et strontium, vis-à-vis des hydrocarbures aromatiques à 8 atomes de carbone.

De manière surprenante, il apparaît que de nouveaux adsorbants à base de zéolithe X comprenant du baryum, du potassium, du strontium et du sodium et présentant une composition particulière en baryum, potassium, strontium et sodium, permettent de manière concomitante de maximiser la productivité et minimiser les coûts de production d'un procédé de séparation du *para*-xylène contenu dans les coupes aromatiques en C8. La présente invention propose également un procédé de séparation des xylènes mettant en oeuvre un adsorbant à base de zéolithe X présentant une composition particulière en baryum, potassium, strontium et sodium, permettant la production de *para*-xylène à haute pureté avec une productivité améliorée à partir d'une charge d'hydrocarbures aromatiques contenant des isomères à 8 atomes de carbone.

RESUME DE L'INVENTION

L'invention concerne un adsorbant zéolithique comprenant des cristaux de zéolithe X et comprenant du baryum, du potassium, du strontium et du sodium, dans lequel le ratio molaire $K_2O / (K_2O + SrO + BaO + Na_2O)$ des espèces sous forme d'oxydes est compris entre 1,5 % et 8,0 % bornes incluses et le ratio molaire $SrO / (K_2O + SrO + BaO + Na_2O)$ des espèces sous forme d'oxydes est compris entre 0,5 % et 8 %, bornes incluses, de préférence les cristaux de zéolithe X ayant un rapport atomique Si/Al compris entre 1,00 et 1,50 bornes incluses. Dans une variante avantageuse, le ratio molaire en $K_2O / (K_2O + SrO + BaO + Na_2O)$ des espèces sous forme d'oxydes est compris entre 2,0 % et 7,0

%, de préférence entre 2,5 et 6,0 %, de manière très préférée entre 2,5% et 4,0%, bornes incluses, par exemple égal à 3,0%. Le ratio molaire SrO / ($K_2O$ + SrO + BaO + $Na_2O$) des espèces sous forme d'oxydes est de préférence compris entre 0,5 % et 7,0%, de manière très préférée entre 1,0% et 6,0%, et de manière encore plus préférée entre 1,0% et 4,0 % bornes incluses. Dans une variante avantageuse, le ratio molaire en SrO / ($K_2O$ + SrO +BaO + $Na_2O$) des espèces sous forme d'oxydes est compris entre 1,0 et 3,0%, par exemple 1,5%.

De préférence, le ratio molaire SrO / $K_2O$ des espèces sous forme d'oxydes est compris entre 0,3 et 2,0, de préférence entre 0,35 et 1,5, de manière préférée entre 0,4 et 1,0 bornes incluses. Dans une variante avantageuse, le ratio molaire SrO / $K_2O$ des espèces sous forme d'oxydes est par exemple égal à 0,5.

La teneur en oxyde de sodium $Na_2O$ est avantageusement inférieure à 0,3% poids et de manière préférée inférieure à 0,2% poids par rapport la masse totale de l'adsorbant. La teneur totale en oxydes d'ions alcalins ou alcalino-terreux autres que l'oxyde de baryum BaO, l'oxyde de potassium $K_2O$, l'oxyde de strontium SrO et l'oxyde de sodium $Na_2O$ est avantageusement inférieure à 1% poids, de préférence comprise entre 0 et 0,5% poids, et de manière très préférée entre 0 et 0,3% en poids, bornes incluses, par rapport à la masse totale de l'adsorbant.

La somme des teneurs en oxyde de baryum BaO, en oxyde de potassium K2O, en oxyde de strontium SrO, en oxyde de sodium Na2O et en éventuels autres oxydes alcalins ou alcalino terreux représente la teneur totale en % poids des espèces sous forme d'oxydes présentes dans l'adsorbant. La teneur en oxyde de baryum en % poids par rapport à la masse totale de l'adsorbant peut donc être calculée directement à partir des autres teneurs des espèces sous forme d'oxydes en % poids par rapport à la masse totale de l'adsorbant.

Dans la présente invention, il doit être compris que les teneurs pondérales exprimées en poids d'oxydes sont exprimées par rapport au poids total de l'adsorbant anhydre (poids corrigé de la perte au feu).

Les cristaux de zéolithes X ont avantageusement un rapport atomique Si/Al compris entre 1,00 et 1,50, de préférence entre 1,00 et 1,45, de préférence encore entre 1,05 et 1,50, plus préférentiellement entre 1,05 et 1,45, et de manière encore préférée entre 1,10 et 1,50, et de manière encore plus préférée entre 1,10 et 1,45, bornes incluses.

Le diamètre moyen en nombre des cristaux de zéolithes X est avantageusement inférieur ou égal à 1,5 $\mu$m, de préférence compris entre 0,1 $\mu$m et 1,2 $\mu$m, de manière plus préférée compris entre 0,1 $\mu$m et 1,0 $\mu$m, bornes incluses.

La perte au feu de l'adsorbant selon l'invention, mesurée à 950 °C selon la norme NF EN 196-2 est avantageusement comprise entre 4,0 et 7,7% et de préférence entre 4,5 et 6,5 % et de manière très préférée entre 4,8 et 6,0 % poids, bornes incluses.

Le diamètre moyen en nombre de l'adsorbant selon l'invention peut être compris entre 0,2 mm et 2,0 mm, en particulier entre 0,2 mm et 0,8 mm et de préférence entre 0,2 mm et 0,65 mm, bornes incluses.

L'invention concerne également un procédé de préparation d'un adsorbant tel que décrit ci-dessus, comprenant au moins les étapes de :

a) agglomération d'une poudre de cristaux de zéolithe X avec un liant, et mise en forme, puis séchage et calcination,
b) zéolithisation éventuelle du liant,
c) échange cationique de l'aggloméré, de manière simultanée, séquencée ou alternée par mise en contact avec des solutions contenant les ions baryum, les ions potassium ou les ions strontium seuls ou en mélange, en une ou plusieurs fois, puis lavage et séchage de l'aggloméré ainsi traité, et
d) activation de l'adsorbant zéolithique ainsi obtenu.

De préférence, le procédé de préparation de l'adsorbant met en oeuvre une étape b) de zéolithisation du liant.

De préférence, la ou les solutions d'ions baryum, d'ions strontium, d'ions potassium de l'étape c) ont une concentration comprise entre 0,5 et 2M.

L'invention concerne également l'utilisation dudit adsorbant selon l'invention dans les procédés de :

• séparation de coupes d'isomères aromatiques en C8 et notamment des xylènes,
• séparation d'isomères de toluène substitué tels que nitrotoluène, diéthyltoluène, toluènediamine, et autres,
• séparation des crésols,
• séparation des alcools polyhydriques,

et notamment pour la séparation de *para*-xylène à partir de coupes d'isomères aromatiques à 8 atomes de carbone.

L'invention concerne également un procédé de récupération de *para*-xylène à partir de coupes d'isomères d'hydrocarbures aromatiques contenant 8 atomes de carbone, en phase liquide ou en phase gazeuse, par adsorption du *para*-xylène, comprenant les étapes successives suivantes:

a) une étape de mise en contact de la charge avec un lit d'adsorbant comprenant au moins dudit adsorbant zéolithique selon l'invention,
b) une étape de mise en contact du lit d'adsorbant avec un désorbant,

de préférence choisi parmi le toluène et le *para*-diéthylbenzène.

Ledit procédé peut être de type lit mobile simulé, de préférence à contre-courant simulé.

Dans ce qui va suivre, et à moins d'une autre indication, les bornes d'un domaine de valeurs sont comprises dans ce domaine, notamment dans les expressions « compris entre » et « allant de ... à ... ».

DESCRIPTION DETAILLEE DE L'INVENTION

La présente invention a ainsi pour premier objet des adsorbants zéolithiques à base de zéolithe X. Ces adsorbants sont particulièrement adaptés pour une utilisation dans un procédé de séparation du *para*-xylène en phase liquide, de préférence de type contre-courant simulé.

Ainsi, la présente invention concerne un adsorbant zéolithique comprenant des cristaux de zéolithe X et comprenant du baryum, du strontium, du potassium et du sodium, dans lequel le ratio molaire $K_2O$ / ($K_2O$ + SrO + BaO + $Na_2O$) des espèces sous forme d'oxydes est compris entre 1,5% et 8,0%, bornes incluses et le ratio molaire SrO / ($K_2O$ + SrO + BaO + $Na_2O$) des espèces sous forme d'oxydes est compris entre 0,5 % et 8 % bornes incluses, de préférence les cristaux de zéolithe X ayant un rapport atomique Si/Al compris entre 1,00 et 1,60 bornes incluses. Dans une variante avantageuse, le ratio molaire en $K_2O$ / ($K_2O$ + SrO + BaO + $Na_2O$), exprimé en termes d'oxydes, est compris entre 2,0% et 7,0%, et de manière préférée entre 2,5% et 6,0%, de manière très préférée entre 2,5 et 4,0% bornes incluses, par exemple égal à 3,0%. Le ratio molaire SrO / ($K_2O$ + SrO + BaO + $Na_2O$) des espèces sous forme d'oxydes est de préférence compris entre 0,5 % et 7,0%, de manière très préférée entre 1,0% et 6,0%, et de manière encore plus préférée entre 1,0 % et 4,0% bornes incluses. Dans une variante avantageuse, le ratio molaire en SrO / ($K_2O$ + SrO +BaO + $Na_2O$) des espèces sous forme d'oxydes est compris entre 1,0 et 3,0%, bornes incluses, par exemple 1,5%.

De préférence, le ratio molaire SrO / $K_2O$ des espèces sous forme d'oxydes est compris entre 0,3 et 2,0, de préférence entre 0,35 et 1,5, de manière préférée entre 0,4 et 1,0, bornes incluses. Dans une variante avantageuse, le ratio molaire SrO / $K_2O$ des espèces sous forme d'oxydes est par exemple égal à 0,5.

Les adsorbants selon l'invention peuvent également comprendre une phase non zéolithique, c'est-à-dire une phase non cristalline qui est essentiellement inerte vis-à-vis de l'adsorption. Dans le cas où l'adsorbant selon l'invention comprend une phase non zéolithique, le ratio molaire $K_2O$ / ($K_2O$ + SrO + BaO + $Na_2O$) prend en compte les oxydes compris dans ladite phase non zéolithique.

La teneur en oxyde de sodium $Na_2O$ dans l'adsorbant selon l'invention est avantageusement inférieure à 0,3% poids et de manière préférée inférieure à 0,2% poids par rapport à la masse totale de l'adsorbant. La teneur totale en oxydes d'ions alcalins ou alcalino-terreux autres que l'oxyde de baryum BaO, l'oxyde de potassium $K_2O$, l'oxyde de strontium SrO et l'oxyde de sodium $Na_2O$ dans l'adsorbant selon l'invention est avantageusement inférieure à 1% poids, de préférence entre 0 et 0,5% poids, et de manière très préférée entre 0 et 0,3% en poids, bornes incluses, par rapport à la masse totale de l'adsorbant.

L'adsorbant zéolithique selon la présente invention est un adsorbant à base de cristaux de zéolithe FAU de type X. Par « zéolithe X », on entend les zéolithes dont le ratio atomique Si/Al est compris entre 1,00 et 1,50 bornes incluses, de préférence entre 1,05 et 1,50 bornes incluses et de manière encore plus préférée entre 1,10 et 1,50 bornes incluses.

Parmi les zéolithes X, il est maintenant communément admis de reconnaître deux sous-groupes dénommés zéolithes LSX et zéolithes MSX. Les zéolithes LSX présentent un ratio atomique Si/Al égal à environ 1 et les zéolithes MSX présentent un ratio atomique Si/Al compris entre environ 1,05 et environ 1,15, bornes incluses.

Dans l'adsorbant zéolithique de la présente invention, et selon un mode de réalisation préféré, par « zéolithe FAU de type X », on entend les zéolithes FAU de type X définies ci-dessus, ces dites zéolithes étant à porosité hiérarchisée c'est-à-dire, les zéolithes de type X à porosité hiérarchisée (ou zéolithe XPH), les zéolithes de type MSX à porosité hiérarchisée (ou MSXPH) et les zéolithes de type LSX à porosité hiérarchisée (ou LSXPH), et plus particulièrement les zéolithes FAU à porosité hiérarchisée et de rapport atomique Si/Al compris entre 1,00 et 1,50, de préférence entre 1,00 et 1,45, de préférence encore entre 1,05 et 1,50, plus préférentiellement entre 1,05 et 1,45 et de manière encore préférée entre 1,10 et 1,50, et de manière encore plus préférée entre 1,10 et 1,45, bornes incluses.

Par « zéolithe à porosité hiérarchisée », on entend une zéolithe possédant à la fois des micropores et des mésopores, autrement dit une zéolithe à la fois microporeuse et mésoporeuse. Par « zéolithe mésoporeuse », on entend une zéolithe dont les cristaux zéolithiques microporeux présentent, conjointement à la microporosité, des cavités internes de taille nanométrique (mésoporosité), facilement identifiables par observation au moyen d'un Microscope Électronique à Transmission (MET ou « TEM » en langue anglaise), comme décrit par exemple dans US 7 785 563 : l'observation par microscopie électronique à transmission (MET) permet de vérifier si les cristaux zéolithiques sont des cristaux de zéolithe pleins (i.e. non mésoporeux) ou des agrégats de cristaux de zéolithes pleins ou des cristaux mésoporeux ou des agrégats de cristaux mésoporeux.

La structure cristalline de la zéolithe FAU de type X dans l'adsorbant zéolithique de la présente invention, est identifiable par diffraction des rayons X (connue de l'homme du métier sous l'acronyme DRX).

Selon un mode de réalisation préféré, l'adsorbant zéolithique présente un rapport atomique Si/Al compris entre 1,00 et 2,00 de préférence entre 1,00 et 1,80 bornes incluses, de préférence encore entre 1,15 et 1,80, bornes incluses et de manière encore plus préférée entre 1,15 et 1,60 bornes incluses.

Dans le présent document, on emploie l'appellation « diamètre moyen en nombre » ou bien « taille » pour les cristaux de zéolithe et pour les agglomérés zéolithiques. La méthode de mesure de ces grandeurs est explicitée plus loin dans la description. Selon un mode de réalisation préféré de la présente invention, le diamètre moyen en nombre des cristaux de zéolithes X est inférieur ou égal à 1,5 $\mu$m, de préférence compris entre 0,1 $\mu$m et 1,2 $\mu$m, de manière plus préférée compris entre 0,1 $\mu$m et 1,0 $\mu$m, bornes incluses.

L'adsorbant zéolithique de l'invention est de préférence sous la forme d'un aggloméré, c'est-à-dire qu'il est constitué de cristaux de zéolithe(s) et d'au moins une phase non zéolithique qui est un liant d'agglomération permettant la cohésion des cristaux entre eux. Ainsi l'adsorbant zéolithique de l'invention est souvent dénommé « aggloméré » dans le présent exposé.

La fraction massique de zéolithe X dans l'adsorbant selon la présente invention peut être d'au moins 80% poids de zéolithe(s) X par rapport au poids total de l'adsorbant anhydre, de préférence d'au moins 90%, cette fraction massique pouvant aller jusqu'à 100% et typiquement jusqu'à 99,5% poids.

Selon un mode de réalisation préféré, l'adsorbant zéolithique selon l'invention présente une perte au feu mesurée à 950 °C selon la norme NF EN 196-2 comprise entre 4,0% et 7,7%, préférence entre 4,5 et 6,5 % et avantageusement entre 4,8 et 6%, bornes incluses.

L'adsorbant zéolithique selon la présente invention présente préférentiellement une résistance mécanique généralement supérieure ou égale à 1,8 MPa, typiquement supérieure ou égale à 2,1 MPa. Cette résistance mécanique est mesurée par la méthode Shell série SMS1471-74 adaptée pour des agglomérés de taille inférieure à 1,6 mm.

La capacité d'adsorption est quant à elle mesurée par mesure du volume microporeux de l'adsorbant évalué d'après l'équation de Dubinin-Raduskevitch par adsorption d'azote ($N_2$) à une température de 77K, après dégazage sous vide à 300°C pendant 16 heures. Le volume microporeux des adsorbants zéolithiques de l'invention a ainsi été mesuré comme étant compris entre 0,245 cm$^3$/g et 0,300 cm$^3$/g, typiquement dans une plage allant de 0,250 cm$^3$/g à 0,290 cm$^3$/g, bornes incluses.

Selon un autre aspect, l'invention concerne un procédé de préparation des agglomérés zéolithiques tels qu'ils viennent d'être définis, procédé qui comprend au moins les étapes de :

a) agglomération d'une poudre de cristaux de zéolithe X avec un liant, et mise en forme, puis séchage et calcination,
b) zéolithisation éventuelle dudit liant, de préférence par action d'une solution basique alcaline,
c) échange cationique de l'aggloméré par mise en contact avec des solutions d'ions baryum, d'ions potassium ou d'ions strontium. Les concentrations en ions potassium, en ions strontium ou en ions baryum dans la solution sont adaptées afin d'atteindre les teneurs en baryum, potassium et strontium visées dans l'adsorbant et donc les ratios molaires $K_2O$ / ($K_2O$ + $SrO$ + $BaO$ + $Na_2O$) et $SrO$ / ($K_2O$ + $SrO$ + $BaO$ + $Na_2O$) visés. L' échange cationique de l'aggloméré au baryum , potassium et strontium peut être réalisé de manière simultanée, séquencée ou alternée par mise en contact avec des solutions contenant les ions baryum, les ions potassium ou les ions strontium seuls ou en mélange de 2 ou 3 ions.

Ces opérations peuvent être réalisées une ou plusieurs fois.

Entre chaque étape d'échange, le solide est lavé plusieurs fois de manière à le débarrasser des excédents de sel et séché à la fin des différentes étapes d'échange.

d) activation de l'aggloméré zéolithique.

De préférence, le procédé de préparation des agglomérés zéolithiques met en oeuvre une étape b) de zéolithisation du liant.

De préférence, la ou les solutions d'ions baryum, d'ions strontium, d'ions potassium de l'étape c) ont une concentration comprise entre 0,5 et 2M.

La taille des cristaux de zéolithe X utilisés à l'étape a) est mesurée par observation au microscope électronique à balayage (MEB) ou par observation au microscope électronique en transmission (MET). Cette observation MEB ou MET permet également de confirmer la présence de phase non zéolithique comprenant par exemple le liant ou le liant résiduel non converti lors de l'étape optionnelle de zéolithisation ou toute autre phase amorphe dans les agglomérés.

Selon un mode de réalisation, la zéolithe X utilisée à l'étape a) est une zéolithe FAU de type X à porosité hiérarchisée. Les cristaux de zéolithe FAU de type X à porosité hiérarchisée présentant une importante surface externe peuvent être obtenus selon diverses méthodes connues de l'homme du métier et par exemple selon la synthèse décrite par Inayat et coll. (Angew. Chem. Int. Ed., (2012), 51, 1962-1965).

Il est également possible de préparer lesdits cristaux par synthèse par ensemencement et/ou par ajustement des conditions opératoires de synthèse tels que le rapport $SiO_2/Al_2O_3$, la teneur en sodium et l'alcalinité du mélange de synthèse ou encore selon des procédés de post-traitement de cristaux de zéolithe FAU de type X conventionnels et connus de l'homme du métier.

Les procédés de post-traitements consistent généralement à éliminer des atomes du réseau zéolithique déjà formé, soit par un ou plusieurs traitements acides qui désaluminent le solide, traitement(s) suivi(s) par un ou plusieurs lavage(s) à la soude (NaOH) afin d'éliminer les résidus aluminiques formés, comme décrit par exemple par D. Verboekend et coll. (Adv. Funct. Mater., 22, (2012), pp. 916-928), soit encore par des traitements qui associent l'action d'un acide et celle d'un agent structurant qui améliorent l'efficacité du traitement acide, comme décrit par exemple dans la demande WO2013/106816.

L'agglomération et la mise en forme (étape a) peuvent être réalisées selon toutes les techniques connues de l'homme de l'art, telles qu'extrusion, compactage, agglomération, et autres. Les proportions de liant d'agglomération, éventuellement zéolithisable, (voir définition plus loin) et de zéolithe(s) mises en oeuvre sont typiquement celles de l'art antérieur, c'est-à-dire de 5 parties à 20 parties en poids de liant pour 95 parties à 80 parties en poids de zéolithe. Les agglomérés issus de l'étape a), qu'ils soient sous forme de billes, d'extrudés ou autres, ont en général un diamètre moyen en nombre (ou leur plus grande dimension lorsqu'ils ne sont pas sphériques) compris entre 0,2 mm et 2 mm, et en particulier comprise entre 0,2 mm et 0,8 mm et de préférence entre 0,2 mm et 0,65 mm, bornes incluses.

À l'issue de l'étape a), les particules d'agglomérés les plus fines peuvent être éliminées par cyclonage et/ou tamisage et/ou les particules trop grosses par tamisage ou concassage, dans le cas d'extrudés, par exemple.

Le liant d'agglomération mis en œuvre à l'étape a) peut être zéolithisable. Il contient alors au moins 80 % de préférence, au moins 90%, de préférence encore au moins 95%, plus particulièrement au moins 96%, en poids, d'argile zéolithisable et peut également contenir d'autres liants minéraux tels que bentonite, attapulgite, et autres. Par argile zéolithisable, on entend une argile ou un mélange d'argiles qui sont susceptibles d'être converties en matière zéolithique (c'est-à-dire matière active au sens de l'adsorption), le plus souvent par action d'une solution basique alcaline. L'argile zéolithisable appartient en général à la famille des kaolins, kaolinites, nacrites, dickites, halloysite et/ou métakaolins. Le kaolin est préféré et le plus couramment utilisé.

D'autres argiles telles que notamment la sépiolite ou l'attapulgite peuvent également être utilisées.

Dans tous les cas, les argiles peuvent être utilisées dans leur état brut ou peuvent être préalablement soumises à un ou plusieurs traitements, par exemple choisis parmi calcination, traitement à l'acide, modification chimique, et autres.

La poudre de zéolithe X mise en oeuvre à l'étape a) peut être issue de la synthèse de cristaux de zéolithe X comprenant majoritairement, voir exclusivement des cations sodium, par exemple les zéolithes NaX, mais on ne sortirait pas du cadre de l'invention en utilisant une poudre ayant subi un ou plusieurs échanges cationiques, après sa synthèse et avant sa mise en oeuvre à l'étape a).

Lors de l'étape a), outre la poudre de zéolithe X et le liant, un ou plusieurs additifs peuvent également être ajoutés, par exemple des additifs destinés à faciliter l'agglomération ou à améliorer le durcissement des agglomérés formés tels que la lignine, l'amidon, la carboxyméthylcellulose, et autres additifs connus de l'homme du métier. De la silice peut également être ajoutée. La source éventuelle de silice peut être de tout type connu de l'homme du métier, spécialiste de la synthèse de zéolithes, par exemple de la silice colloïdale, des diatomées, de la perlite, des cendres de calcination (« fly ash » en langue anglaise), du sable, ou toute autre forme de silice solide.

Après le séchage à l'étape a), la calcination est menée à une température en général comprise entre 500°C et 700°C. Dans le cas où la mise en forme est réalisée avec une argile zéolithisable, cette étape permet de transformer l'argile zéolithisable, typiquement le kaolin, en métakaolin qui peut après être converti en zéolithe lors de l'étape de zéolithisation (étape b)). Le principe en est exposé dans « Zeolite Molecular Sieves » de D.W. Breck, John Wiley and Sons, New York, (1973), p. 314-315.

La zéolithisation du liant d'agglomération est pratiquée selon toute méthode connue de l'homme du métier et peut par exemple être réalisée par immersion du produit de l'étape a) dans une solution basique alcaline, en général aqueuse, par exemple une solution aqueuse d'hydroxyde de sodium et/ou d'hydroxyde de potassium.

En règle générale, la concentration de la solution alcaline de zéolithisation est de préférence comprise entre 0,5 M et 5 M, plus particulièrement entre 0,5 et 2M. La zéolithisation s'opère de préférence à chaud, à une température supérieure à la température ambiante, et typiquement à des températures de l'ordre de 80°C à 100°C, par exemple comprises entre la température ambiante (soit environ 20°C) et la température d'ébullition de la solution alcaline de zéolithisation. La durée du processus de zéolithisation est généralement comprise entre quelques dizaines de minutes et quelques heures, de préférence entre environ 1 heure et 8 heures.

L'étape c) d'échange au baryum et/ou strontium et/ou au potassium des cations de la zéolithe X s'effectue selon les méthodes classiques connues de l'homme du métier, et le plus souvent par mise en contact des agglomérés issus de l'étape a) ou de l'étape b) avec un sel, tel que le chlorure de baryum ($BaCl_2$) pour l'échange au baryum et/ou le chlorure de strontium ($SrCl_2$) pour l'échange au strontium et/ou le chlorure de potassium (KCl) pour l'échange au potassium, en solution aqueuse à une température comprise entre la température ambiante et 100°C, et de préférence comprise entre 80°C et 100°C. Pour obtenir rapidement des teneurs en oxyde de sodium faibles, i.e. inférieures à 1%, on préfère opérer avec un large excès d'ions baryum et/ou strontium et/ou potassium par rapport aux cations de la zéolithe que l'on souhaite échanger, typiquement un excès de l'ordre de 10 à 12, avantageusement en procédant par échanges successifs. De préférence, la ou les solutions d'ions baryum, d'ions strontium, d'ions potassium de l'étape c) ont une concentration comprise entre 0,5 et 2M.

Comme indiqué précédemment, il est également possible d'agglomérer à l'étape a) de la poudre de zéolithe X contenant déjà des ions potassium (pré-échange des cations présents dans la zéolithe X de départ, typiquement cations sodium, par des ions potassium avant l'étape a)) et s'affranchir ou non des échanges au potassium lors de l'étapes c).

On procède ensuite à un lavage, généralement et de préférence à l'eau, puis d'un séchage de l'aggloméré ainsi obtenu.

L'activation qui suit le séchage, est conduite de manière classique, selon les méthodes connues de l'homme du métier, par exemple à une température en général comprise entre 100°C et 400°C, de préférence entre 200°C et 300°C. Cette étape d) d'activation a pour but de fixer la teneur en eau, ainsi que la perte au feu de l'adsorbant de façon optimale pour l'utilisation envisagée. On procède en général par activation thermique qu'on exécute préférentiellement entre 200°C et 300°C pendant une durée déterminée en fonction de la teneur en eau et de la perte au feu souhaitées, typiquement de 1 à 6 heures.

La présente invention concerne également les utilisations des adsorbants zéolithiques décrits ci-dessus comme agents d'adsorption susceptibles de remplacer avantageusement les agents d'adsorption décrits dans la littérature pour les utilisations listées ci-dessous :

- séparation de coupes d'isomères aromatiques en C8 et notamment des xylènes,
- séparation d'isomères de toluène substitué tels que nitrotoluène, diéthyltoluène, toluènediamine, et autres,
- séparation des crésols,
- la séparation des alcools polyhydriques, tels que les sucres.

L'invention concerne notamment un procédé de récupération de *para*-xylène à haute pureté à partir de coupes d'isomères aromatiques à 8 atomes de carbone consistant à utiliser, comme agent d'adsorption du *para*-xylène, un adsorbant zéolithique selon l'invention, mis en œuvre dans des procédés en phase liquide mais aussi en phase gazeuse. Par *para*-xylène de haute pureté, nous entendons un produit approprié pour une utilisation dans la production d'acide téréphtalique ou de téréphtalate de diméthyle, c'est à dire une pureté d'au moins 99,5% poids, de préférence au moins 99,7% poids, de préférence au moins 99,8% poids et plus préférablement encore au moins 99,9% poids. La pureté du *para*-xylène peut-être déterminée par des méthodes chromatographiques. Une méthode de chromatographie en phase gazeuse utilisable à la fois pour la détermination de la pureté du *para*-xylène et des quantités spécifiques d'impuretés est la méthode ASTM D-3798.

On peut ainsi séparer le produit désiré (*para*-xylène) par chromatographie liquide d'adsorption préparative (en batch), et avantageusement en continu en lit mobile simulé, c'est-à-dire à contre-courant simulé ou à co-courant simulé, et plus particulièrement à contre-courant simulé.

Le procédé de récupération de *para*-xylène selon l'invention utilisant l'adsorbant décrit selon l'invention présente l'avantage de maximiser la productivité mais également de minimiser les coûts opératoires du procédé, c'est-à-dire à la fois de maximiser le débit de charge à traiter et de minimiser le débit de désorbant nécessaire. Ceci est particulièrement vrai dans les conditions opératoires d'unité industrielle d'adsorption de type contre-courant simulé suivantes :

- nombre de lits : 6 à 30,
- nombre de zones : au moins 4 zones de fonctionnement, chacune étant localisées entre un point d'alimentation et un point de soutirage,
- température comprise entre 100°C et 250°C, de préférence entre 150°C et 190°C,
- pression de l'unité industrielle comprise entre la pression de bulle des xylènes à la température du procédé et 3 MPa,
- rapport des débits désorbant/charge compris entre 0,7 et 2,5, par exemple entre 0,9 et 1,8 pour une unité d'adsorption seule (stand alone) et entre 0,7 et 1,4 pour une unité d'adsorption combinée à une unité de cristallisation,
- taux de recyclage (i.e. ratio du débit de recyclage moyen (moyenne des débits de zones pondérée du nombre de lits par zones) sur le débit de charge) compris entre 2,5 et 12, de préférence entre 3,5 et 6.

L'invention porte également sur un procédé de récupération de para-xylène à partir de coupes d'isomères d'hydrocarbures aromatiques contenant 8 atomes de carbone, en phase liquide, par adsorption du para-xylène , comprenant les étapes successives suivantes :

a) une étape de mise en contact de la charge avec un lit d'adsorbant comprenant au moins un adsorbant zéolithique tel que défini ci-dessus,
b) une étape de mise en contact du lit d'adsorbant avec un désorbant, de préférence choisi parmi le toluène et le para-diéthylbenzène.

Le procédé de récupération de *para*-xylène peut être de type lit mobile simulé, de préférence à contre-courant simulé.

L'invention porte également sur un procédé de récupération de *para*-xylène à partir de coupes d'isomères d'hydrocarbures aromatiques contenant 8 atomes de carbone, en phase gazeuse, par adsorption du *para*-xylène au moyen d'un adsorbant tel que défini ci-dessus, comprenant les étapes successives suivantes :

a) une étape de mise en contact de la charge avec un lit d'adsorbant comprenant au moins un adsorbant zéolithique tel que défini ci-dessus,

b) une étape de mise en contact du lit d'adsorbant avec un désorbant, de préférence choisi parmi le toluène et le para-diéthylbenzène.

Dans une variante, le procédé de séparation du paraxylène à haute pureté est réalisé en lit mobile simulé à partir d'une charge d'hydrocarbures aromatiques contenant des isomères à 8 atomes de carbones comprenant les étapes suivantes :

a) une étape de mise en contact de la charge avec un lit d'adsorbant comprenant au moins un adsorbant zéolithique tel que défini précédemment, de manière à adsorber préférentiellement le paraxylène,

b) une étape de mise en contact du lit d'adsorbant avec un désorbant, le désorbant étant préférentiellement soit du toluène, soit du paradiéthylbenzène,

c) une étape de soutirage du lit d'adsorbant d'un flux contenant le désorbant et les produits de la charge les moins sélectivement adsorbés,

d) une étape de soutirage du lit d'adsorbant d'un flux contenant le désorbant et le produit recherché, à savoir le paraxylène,

e) une séparation du flux issu de l'étape c) en un premier flux contenant le désorbant et un second flux contenant les produits de la charge les moins sélectivement adsorbés,

f) une séparation du flux issu de l'étape d) en un premier flux contenant le désorbant et un second flux contenant du paraxylène à un niveau de pureté supérieure ou égale à 90%, de manière préférée supérieure ou égale à 99%, et de manière très préférée supérieure ou égale à 99,7%.

On pourra sur ce sujet se référer à l'enseignement des brevets US 2 985 589, US 5 284 992 et US 5 629 467.

Les conditions opératoires d'une unité industrielle d'adsorption à co-courant simulé sont en général les mêmes que celles fonctionnant à contre-courant simulé, à l'exception du taux de recyclage qui est en général compris entre 0,8 et 7. On pourra sur cet aspect se référer aux brevets US 4 402 832 et US 4 498 991.

Le solvant de désorption peut être tout désorbant connu de l'homme du métier et dont le point d'ébullition est inférieur à celui de la charge, tel que le toluène mais aussi un désorbant dont le point d'ébullition est supérieur à celui de la charge, tel que le *para*-diéthylbenzène (PDEB). La sélectivité des adsorbants selon l'invention pour l'adsorption du *para*-xylène contenu dans des coupes aromatiques en C8 est optimale lorsque leur perte au feu mesurée à 950°C est comprise en général entre 4,0% et 7,7%, et de préférence entre 4,5% et 6,5%, et de manière très préférée entre 4,8% et 6,0% bornes incluses.

TECHNIQUES DE CARACTERISATION

**Granulométrie des cristaux** :

L'estimation du diamètre moyen en nombre des cristaux de zéolithe X utilisées à l'étape a) et des cristaux de zéolithe X contenue dans les agglomérés est réalisée par observation au microscope électronique à balayage (MEB) ou par observation au microscope électronique en transmission (MET).

Afin d'estimer la taille des cristaux de zéolithe sur les échantillons, on effectue un ensemble de clichés à un grossissement d'au moins 5000. On mesure ensuite le diamètre d'au moins 200 cristaux à l'aide d'un logiciel dédié, par exemple le logiciel Smile View de l'éditeur LoGraMi. La précision est de l'ordre de 3%.

**Analyse chimique des adsorbants zéolithiques** - **rapports Si/Al et teneurs en oxydes**

Une analyse chimique élémentaire du produit final obtenu à l'issue des étapes a) à d) décrites précédemment, peut être réalisée selon différentes techniques analytiques connues de l'homme du métier. Parmi ces techniques, on peut citer la technique d'analyse chimique par fluorescence de rayons X telle que décrite dans la norme NF EN ISO 12677 : 2011 sur un spectromètre dispersif en longueur d'onde (WDXRF), par exemple Tiger S8 de la société Bruker.

La fluorescence X est une technique spectrale non destructive exploitant la photoluminescence des atomes dans le domaine des rayons X, pour établir la composition élémentaire d'un échantillon. L'excitation des atomes généralement par un faisceau de rayons X ou par bombardement avec des électrons, génère des radiations spécifiques après retour à l'état fondamental de l'atome. Le spectre de fluorescence X a l'avantage de dépendre très peu de la combinaison chimique de l'élément, ce qui offre une détermination précise, à la fois quantitative et qualitative. On obtient de manière classique après étalonnage pour chaque oxyde une incertitude de mesure inférieure à 0,4% en poids. Dans la présente invention les teneurs en baryum, en strontium, en potassium, en silicium et en aluminium sont de préférence mesurées par la méthode fluorescence X décrite ci-dessus.

En revanche pour les éléments plus légers en termes poids atomique tels que le sodium, présents dans l'adsorbant, on préfère pour plus de précision la spectrométrie d'émission atomique avec plasma induit par haute fréquence (ICP-OES pour Inductively Coupled Plasma-Optical Emission Spectroscopy selon la terminologie anglo-saxonne) selon la norme UOP 961-12.

L'ICP est une méthode d'analyse par spectrométrie d'émission atomique dont la source est un plasma généré par couplage inductif. Dans la présente invention les teneurs en sodium sont de préférence mesurées par la méthode ICP selon la norme UOP 961-12. On obtient dans ce cas pour le sodium une incertitude sur la mesure inférieure à 0,01% pour la teneur en poids de l'oxyde de sodium dans l'adsorbant.

Ces analyses chimiques élémentaires permettent à la fois de vérifier le rapport atomique Si/Al de la zéolithe au sein de l'aggloméré, et de vérifier la qualité de l'échange ionique décrit à l'étape c). Dans la description de la présente invention, l'incertitude de mesure du ratio atomique Si/Al est de 0,05%.

La qualité de l'échange ionique est liée au nombre de mole d'oxyde de sodium, $Na_2O$, restant dans l'aggloméré zéolithique après échange. Plus précisément, le taux d'échange par les ions baryum est déterminé par le ratio entre le nombre de moles d'oxyde de baryum, BaO, et le nombre de moles de l'ensemble (BaO + $K_2O$ +SrO + $Na_2O$). De même, le taux d'échange par les ions potassium et strontium est déterminé respectivement par le ratio entre le nombre de moles d'oxyde de potassium, $K_2O$, et le nombre de moles de l'ensemble (BaO + $K_2O$ +SrO + $Na_2O$) ou par le ratio entre le nombre de moles d'oxyde de strontium, SrO, et le nombre de moles de l'ensemble (BaO + $K_2O$ +SrO + $Na_2O$). BaO, $K_2O$, SrO et $Na_2O$ sont exprimés sous forme d'oxydes. Le taux d'échange total par les ions baryum, potassium et strontium est estimé à partir de la somme des trois taux d'échange précédemment décrits, correspondant au rapport entre la somme du nombre de moles d'oxyde de baryum, d'oxyde de potassium et d'oxyde de strontium (BaO + $K_2O$ + SrO) et le nombre de moles de l'ensemble (BaO + $K_2O$ + SrO + $Na_2O$). Il est à noter que les teneurs en différents oxydes sont donnés, en pourcentage en poids par rapport au poids total de l'adsorbant zéolithique anhydre. Dans la description de la présente invention, l'incertitude de mesure sur le ratio molaire $K_2O$ / ($K_2O$ + BaO + SrO + $Na_2O$) est de 0,3%, et l'incertitude de mesure sur le ratio molaire SrO / ($K_2O$ + SrO + BaO + $Na_2O$) est de 0,3%.

**Granulométrie des adsorbants zéolithiques** :

La détermination du diamètre moyen en nombre des adsorbants zéolithiques obtenus à l'issus de l'étape a) d'agglomération et de mise en forme est effectuée par analyse de la distribution granulométrique d'un échantillon d'aggloméré par imagerie selon la norme ISO 13322-2:2006, en utilisant un tapis roulant permettant à l'échantillon de passer devant l'objectif de la caméra.

Le diamètre moyen en nombre est ensuite calculé à partir de la distribution granulométrique en appliquant la norme ISO 9276-2:2001. Dans le présent document, on emploie l'appellation « diamètre moyen en nombre » ou bien « taille » pour les agglomérés zéolithiques. La précision est de l'ordre de 0,01 mm pour la gamme de taille d'agglomérés de l'invention.

**Résistance mécanique des adsorbants zéolithiques :**

La technique de caractérisation de la résistance mécanique représentative de l'écrasement de l'adsorbant au sein d'un lit ou d'un réacteur est la technique de caractérisation de la résistance mécanique en lit, telle que décrite dans la méthode Shell série SMS1471-74 (Shell Method Series SMS1471-74 Détermination of Bulk Crushing Strength of Catalysts. Compression-Sieve Method"), associée à l'appareil "BCS Tester" commercialisé par la société Vinci Technologies. Cette méthode, initialement destinée à la caractérisation de catalyseurs de 3 à 6 mm est basée sur l'utilisation d'un tamis de 425 μm qui va permettre notamment de séparer les fines créées lors de l'écrasement. L'utilisation d'un tamis de 425 μm reste adaptée pour des particules de diamètre supérieur à 1,6mm, mais doit être adaptée selon la granulométrie des adsorbants zéolithiques que l'on cherche à caractériser. La norme ASTM D7084-04 qui décrit également une méthode de mesure de la résistance à l'écrasement en lit de catalyseurs ("Determination of Bulk Crush Strength of Catalysts and Catalyst Carriers") définit le passage du tamis à utiliser comme étant égal à la moitié du diamètre des particules de catalyseurs à caractériser. La méthode prévoit une étape préliminaire de tamisage de l'échantillon de catalyseurs ou adsorbants à caractériser. Si une quantité égale à 10% pds de l'échantillon passe à travers la grille, un tamis de passage plus petit sera utilisé.

Les agglomérés de la présente invention, généralement sous forme de billes ou d'extrudés, ont en général un diamètre moyen en nombre ou une longueur, i.e. plus grande dimension dans le cas des agglomérés non sphériques, comprise entre 0,2 mm et 2 mm, et en particulier comprise entre 0,2 mm et 0,8 mm et de préférence entre 0,2 mm et 0,65 mm. Par conséquent, un tamis adapté tel que moins de 10% poids de l'échantillon passe à travers la grille lors d'une étape préalable de tamisage est utilisé à la place du tamis de 425 $\mu$m mentionné dans la méthode Shell standard SMS1471-74.

Le protocole de mesure est le suivant : un échantillon de 20 cm$^3$ d'adsorbants agglomérés, préalablement tamisé avec le tamis adapté et préalablement séché à l'étuve pendant au moins 2 heures à 250°C (au lieu de 300°C mentionné dans la méthode Shell standard SMS1471-74), est placé dans un cylindre métallique de section interne connue. Une force croissante est imposée par paliers sur cet échantillon par l'intermédiaire d'un piston, à travers un lit de 5 cm$^3$ de billes d'acier afin de mieux répartir la force exercée par le piston sur les agglomérés d'adsorbants (utilisation de billes de 2 mm de diamètre pour des particules de forme sphérique de diamètre strictement inférieur à 1,6 mm). Les fines obtenues aux différents paliers de pression sont séparées par tamisage (avec tamis adapté) et pesées.

La résistance à l'écrasement en lit est déterminée par la pression en mégaPascal (MPa) pour laquelle la quantité de fines cumulées passant à travers le tamis s'élève à 0,5% pondéral de l'échantillon. Cette valeur est obtenue en traçant sur un graphique la masse de fines obtenue en fonction de la force appliquée sur le lit d'adsorbant et en interpolant à 0,5 % massique de fines cumulées. La résistance mécanique à l'écrasement en lit est typiquement comprise entre quelques centaines de kPa et quelques dizaines de MPa et généralement comprise entre 0,3 MPa et 4 MPa. La précision est de manière classique inférieure à 0,1 MPa.

**Détermination des fractions zéolithiques des adsorbants zéolithiques :**

La nature et la quantité des différentes fractions zéolithiques sont déterminées par analyse par diffraction de rayons X, connue de l'homme du métier sous l'acronyme DRX. Cette analyse est réalisée sur un appareil de la marque Bruker, puis la quantité des fractions zéolithiques est évaluée au moyen du logiciel TOPAS de la société Bruker.

**Volume microporeux :**

La cristallinité des agglomérés est également évaluée par mesure de leur volume microporeux en le comparant à celui d'une référence appropriée (zéolithe 100% cristalline dans des conditions de traitements cationiques identiques ou zéolithe théorique). Ce volume microporeux est déterminé à partir de la mesure de l'isotherme d'adsorption de gaz, tel que l'azote, à sa température de liquéfaction. Préalablement à l'adsorption, l'adsorbant zéolithique est dégazé entre 300°C - 450°C pendant une durée de 9 heures à 16 heures, sous vide (P < 6,7.10$^{-4}$ Pa). La mesure de l'isotherme d'adsorption d'azote à 77K est ensuite effectuée sur un appareil de type ASAP 2010 M de Micromeritics, en prenant au moins 35 points de mesure à des pressions relatives de rapport $P/P_0$ compris entre 0,002 et 1. Le volume microporeux est déterminé selon Dubinin et Raduskevitch à partir de l'isotherme obtenue, en appliquant la norme ISO 15901-3:2007. Le volume microporeux évalué selon Dubinin et Raduskevitch s'exprime en cm$^3$ d'adsorbat liquide par gramme d'adsorbant. L'incertitude de mesure est de $\pm$ 0,003.

**Perte au feu des adsorbants zéolithiques :**

La perte au feu est déterminée en atmosphère oxydante, par calcination de l'échantillon à l'air à une température de 950°C $\pm$ 25°C, comme décrit dans la norme NF EN 196-2 (avril 2006). L'écart type de mesure est inférieur à 0,1%.

**Caractérisation de l'adsorption en phase liquide par perçage :**

La technique utilisée pour caractériser l'adsorption de molécules en phase liquide sur un solide poreux est la technique dite de perçage, décrite par Ruthven dans « Principles of Adsorption and Adsorption Processes » (Chapitres 8 et 9, John Wiley & Sons, 1984) qui définit la technique des courbes de perçage (« breakthrough curves ») comme l'étude de la réponse à l'injection d'un échelon de constituants adsorbables. L'analyse du temps moyen de sortie (premier moment) des courbes de perçage fournit une information sur les quantités adsorbées et permet également d'évaluer les sélectivités, c'est-à-dire le facteur de séparation, entre deux constituants adsorbables. L'injection d'un constituant non adsorbable utilisé comme traceur est conseillée pour l'estimation des volumes non-sélectifs. L'analyse de la dispersion (second moment) des courbes de perçage, permet d'évaluer la hauteur équivalente de plateaux théoriques, basée sur la représentation d'une colonne par un nombre fini de réacteurs hypothétiques idéalement agités (étages théoriques), qui est une mesure directe de la dispersion axiale et de la résistance au transfert de matière du système.

EXEMPLES

**Préparation des adsorbants zéolithiques**

On prépare un mélange homogène et on agglomère 800 g de cristaux de zéolithe NaX selon le mode opératoire décrit dans la demande de brevet FR2 999 098 (synthèse de l'exemple B) avec 105 g de kaolin (exprimés en équivalent calciné) et 45 g de silice colloïdale vendue sous la dénomination commerciale Klebosol®30 (contenant 30% en poids de $SiO_2$ et 0,5% de $Na_2O$) avec la quantité d'eau qui permet l'extrusion du mélange. Les extrudés sont séchés, concassés de manière à récupérer des grains dont le diamètre moyen en nombre est égal à 0,7 mm, puis calcinés à 550°C sous courant d'azote pendant 2 heures.

200 g d'agglomérés obtenus sont placés dans un réacteur en verre muni d'une double enveloppe régulée à une température de 100°C $\pm$ 1°C, puis on ajoute 1,5 L d'une solution aqueuse d'hydroxyde de sodium de concentration 2,5 M et on laisse le milieu réactionnel sous agitation pendant une durée de 4 heures.

On procède ensuite au lavage des agglomérés en 3 opérations successives de lavage à l'eau suivi de la vidange du réacteur. On s'assure de l'efficacité du lavage en mesurant le pH final des eaux de lavage compris entre 10,0 et 10,5.

**Exemple 1** : **Échange cationique au baryum, au potassium et au strontium**

Les cations sodium des agglomérés obtenus sont échangés par des ions baryum potassium et strontium au moyen d'une solution aqueuse à 0,5M de chlorure de potassium, de chlorure de strontium et de chlorure de baryum à 95°C en 4 étapes. Les concentrations de chlorure de potassium, de chlorure de strontium et de chlorure de baryum dans la solution sont adaptées afin d'atteindre les teneurs en baryum, potassium et strontium visées dans l'adsorbant et donc les ratios molaires $K_2O$ / ($K_2O$ + SrO + BaO + $Na_2O$) et SrO / ($K_2O$ + SrO + BaO + $Na_2O$) visés. À chaque étape, le rapport volume de solution sur masse de solide est de 20 mL/g et l'échange est poursuivi pendant 3 heures à chaque fois. Entre chaque échange, le solide est lavé plusieurs fois de manière à le débarrasser des excédents de sel. Les agglomérés sont ensuite séchés à 80°C pendant 2 heures et enfin activés à 250°C pendant 2 heures sous courant d'azote.

La perte au feu mesurée, comme décrit précédemment, est de 5,6% $\pm$ 0,1% pour chaque échantillon. Le taux d'échange en baryum+potassium+strontium des agglomérés calculés à partir des analyses élémentaires des oxydes de baryum, potassium, strontium et de sodium par fluorescence X et ICP comme décrit dans les techniques de caractérisation est de 99,7 $\pm$ 0,2%.

**Exemple 2** : Test de perçage

Un test de perçage (chromatographie frontale) est ensuite réalisé sur les agglomérés obtenus à l'exemple 1 pour évaluer leur efficacité. La quantité d'adsorbant utilisée pour ce test est d'environ 30 g.

Le mode opératoire pour obtenir les courbes de perçage est le suivant :

- Remplissage de la colonne par le tamis et mise en place dans le banc de test.
- Remplissage par un solvant (toluène) à température ambiante.
- Montée progressive à la température d'adsorption sous flux de solvant (2 cm$^3$/min).
- Injection de solvant à 2 cm$^3$/min lorsque la température d'adsorption est atteinte.
- Permutation solvant/charge pour injecter la charge (2 cm$^3$/min).
- L'injection de la charge est ensuite maintenue un temps suffisant pour atteindre l'équilibre thermodynamique.
- Collecte de la recette du perçage dans un flacon unique puis analyse de la composition de la recette par CPG.

La pression est suffisante pour que la charge reste en phase liquide, soit 1 MPa. La température d'adsorption est de 175°C. La composition de la charge utilisée pour les tests est la suivante :

- *Para-xylène* : 18% poids
- *Méta-xylène* : 18% poids
- *Ortho-xylène* : 18% poids
- *Éthylbenzène* : 18% poids
- *Para-diéthylbenzène* : 18% poids
- *Iso-octane* : 10% poids (celui-ci est utilisé comme traceur pour l'estimation des volumes non-sélectifs et n'intervient pas dans la séparation)

Les sélectivités binaires des composés deux à deux, notées sélectivités binaires $\alpha_{i/k}$ sont calculées à partir

des quantités adsorbées $q_i$ et $q_k$ des composés i et k, ces dernières étant déterminées par bilan matière à partir de l'analyse de la composition de la recette du perçage et de la composition de la charge (charge dans laquelle la fraction massique des composés i et k est $y_i$ et $y_k$) :

$$\alpha_{i/k} = \frac{q_i y_k}{q_k y_i}$$

L'évaluation du potentiel de ces adsorbants lors de la mise en oeuvre à contre-courant simulé, est faite en se basant sur la théorie de l'équilibre appliquée aux systèmes multi-constituants à sélectivités constantes telle que décrite par Mazotti, Storti et Morbidelli dans Robust Design of Countercurrent Adsorption Séparation Processes: 2. Multicomponent Systems, AlChE Journal November 1994 Vol. 40, No. 11. En particulier, on se réfère ici à l'équation 8, qui décrit les conditions à satisfaire sur les débits réduits $m_j$ des 4 sections (j=1 à 4) d'une unité de séparation à contre-courant telle que schématisée en Figure 1 de l'article cité pour obtenir une séparation complète.

$$
\begin{aligned}
&\text{Section 1:} && K_{sp} < m_1\delta_1 < +\infty \\
&\text{Section 2:} && K_{wk} < m_2\delta_2 < K_{sk} \\
&\text{Section 3:} && K_{wk} < m_3\delta_3 < K_{sk} \\
&\text{Section 4:} && -\frac{\varepsilon_p \delta_4}{\sigma(1-\varepsilon_p)} < m_4\delta_4 < K_{ww}
\end{aligned}
\qquad (8)
$$

Cette équation 8 fait référence aux adsorptivités $K_i$ des différents constituants, ainsi qu'au paramètre $\delta_j$ de chaque section j défini par l'équation 7 :

$$\delta_j = \sum_{i=1}^{NC} K_i y_i^j \quad (j = 1, \ldots, 4), \qquad (7)$$

Il faut noter ici que par définition la sélectivité binaire $\alpha_{i/k}$ entre les composés i et k est égal au rapport des adsorptivités $K_i / K_k$.

Le débit réduit de chaque section de l'unité est défini comme étant le rapport du débit de la phase liquide sur le débit de la phase adsorbée. L'équation 8 indique quels sont les débits réduits limites pour chaque section. Dans une unité de séparation à contre-courant à 4 sections, le débit de charge correspond à la différence entre le débit en zone 3 et le débit en zone 2, et le débit de désorbant correspond à la différence entre le débit en zone 1 et le débit en zone 4.

Par conséquent, lorsque l'on veut évaluer la productivité maximale qui peut être atteinte avec un adsorbant donné, on cherche à évaluer la quantité maximale de charge, mais également à minimiser les coûts opératoires. Un adsorbant performant est celui qui permet à la fois de maximiser le débit de charge à traiter et de minimiser le débit de désorbant nécessaire.

Pour déterminer la quantité maximale de charge que l'on peut traiter, on évalue la différence entre le débit maximal en zone 3 et le débit minimal en zone 2. On peut comparer les performances en terme de productivité maximale de deux adsorbants en comparant leur débit réduit maximal de charge déterminé à partir des débits réduits des zones 2 et 3, respectivement $m_2$ et $m_3$, selon la relation : $\max(m_{Charge}) = \max(m_3) - \min(m_2)$. Si on considère un système à sélectivités constantes, la composition de la phase liquide qui donne la contrainte la plus forte en zone 2 et en zone 3 est la composition de la phase liquide au point d'injection de la charge dans l'unité. En effet, à partir de ce point la concentration en para-xylène, qui est le composé le plus adsorbé, augmente dans le sens de circulation du solide en zone 2, et diminue dans le sens de circulation du liquide en zone 3. On peut approximer la composition de ce point à la composition de la charge à traiter, et c'est cette composition qui sera utilisée pour évaluer le terme $\delta_2$ et $\delta_3$ de l'équation 8. Les termes $\delta_2$ et $\delta_3$ étant définis par l'équation 7 mentionnée ci-dessus.

Pour chaque adsorbant, ce débit réduit $\max(m_{Charge})$ est calculé à partir des valeurs de sélectivités binaires mesurées expérimentalement.

Pour déterminer la quantité minimale de désorbant à injecter, on évalue la différence entre le débit minimal en zone 1 et le débit maximal en zone 4. On pourra comparer les performances en terme de régénérabilité de deux adsorbants en comparant leur débit réduit minimal de désorbant déterminé à partir des débits réduits des zones 1 et 4, respectivement $m_1$ et $m_4$, selon la relation : $\min(m_{Dés}) = \min(m_1) - \max(m_4)$.

Pour un système à sélectivités constantes, la composition de la phase liquide qui donne la contrainte la plus forte en zone 1 et en zone 4 est la composition de la phase liquide au point d'injection de désorbant dans l'unité. Au niveau de ce point, la phase liquide contient essentiellement du désorbant. Sa composition est utilisée pour évaluer les termes $\delta_1$ et $\delta_4$ de l'équation 8. Les termes $\delta_1$ et $\delta_4$ étant définis par l'équation 7 mentionnée ci-dessus.

Pour chaque adsorbant, le débit réduit min($m_{Dés}$) est calculé à partir des valeurs de sélectivités binaires mesurées expérimentalement.

Le rapport entre max($m_{charge}$) et min($m_{Dés}$) permet d'indiquer la faculté de l'adsorbant à conjointement maximiser la productivité et minimiser les coûts d'opération du procédé de séparation du paraxylène contenu dans les coupes aromatiques en C8.

Le Tableau 1 permet de comparer le rapport de débits réduits entre max($m_{Charge}$) et min($m_{Dés}$) pour différentes formulations d'adsorbants présentant différents ratios molaires des espèces sous forme d'oxydes $K_2O$ / ($K_2O$ + SrO + BaO + $Na_2O$) et SrO / ($K_2O$ + SrO + BaO + $Na_2O$). Le ratio molaire SrO / $K_2O$ des espèces sous forme d'oxydes est également indiqué.

| Formulation | $K_2O$ / ($K_2O$ + SrO + BaO + $Na_2O$) | SrO / ($K_2O$ + SrO + BaO + $Na_2O$) | SrO / $K_2O$ | max($m_{Charge}$)/min ($m_{Dés}$) |
|---|---|---|---|---|
| A (non conforme) | 0 | 0 | --- | 0,84 |
| B (non conforme) | 3 | 0 | | 0,88 |
| C (non conforme) | 5 | 0 | 0 | 0,89 |
| D (non conforme) | 0 | 3 | --- | 0,86 |
| E (non conforme) | 0 | 5 | --- | 0,82 |
| F (non conforme) | 3 | 8,2 | 2,73 | 0,63 |
| G | 3 | 2 | 0,66 | 0,99 |
| H | 3,5 | 1,5 | 0,42 | 1,01 |
| I | 1,8 | 0,75 | 0,42 | 1,05 |

On s'aperçoit que les adsorbants G, H, et I ayant une teneur contrôlée en strontium et en potassium par rapport à l'ensemble des cations conformément à l'invention permettent d'obtenir des rapports entre max($m_{charge}$) et min($m_{Dés}$) optimaux.

En revanche, les adsorbants A, B, C, D et F présentant des teneurs non conformes à l'invention conduisent à l'obtention de valeurs dégradées pour le rapport entre max($m_{charge}$) et min($m_{Dés}$).

Cela met en évidence l'effet bénéfique de l'utilisation des teneurs en Sr et K décrites dans l'invention.

## Revendications

1. Adsorbant zéolithique comprenant des cristaux de zéolithe X et comprenant du baryum, du potassium, du strontium et du sodium, dans lequel le ratio molaire $K_2O$ / ($K_2O$ + SrO + BaO + $Na_2O$) des espèces sous forme d'oxydes est compris entre 1,5 % et 8,0%, bornes incluses et le ratio molaire SrO / ($K_2O$ + SrO + BaO + $Na_2O$) des espèces sous forme d'oxydes est compris entre 0,5 % et 8,0 %, bornes incluses, de préférence les cristaux de zéolithe X ayant un rapport atomique Si/Al compris entre 1,00 et 1,50 bornes incluses.

2. Adsorbant selon la revendication 1, dans lequel le ratio molaire $K_2O$ / ($K_2O$ + SrO + BaO + $Na_2O$) des espèces sous forme d'oxydes est compris entre 2,0 % et 7,0%, de préférence entre 2,5 et 6,0 %, de manière très préférée entre 2,5 % et 4,0 %, bornes incluses.

3. Adsorbant selon l'une des revendications 1 ou 2, dans lequel le ratio molaire SrO / ($K_2O$ + SrO + BaO + $Na_2O$) des

espèces sous forme d'oxydes est compris entre 0,5% et 7,0%, de préférence entre 1,0% et 6%, de manière très préférée entre 1,0 et 4,0%, de manière encore plus préférée entre 1,0 et 3,0%, bornes incluses.

4. Adsorbant selon l'une des revendications précédentes dans lequel le ratio molaire SrO / $K_2O$ des espèces sous forme d'oxydes est compris entre 0,3 et 2,0, de préférence entre 0,35 et 1,5, de manière préférée entre 0,4 et 1,0 bornes incluses.

5. Adsorbant selon l'une des revendications précédentes comprenant en outre une phase non zéolithique.

6. Adsorbant selon l'une des revendications précédentes dans lequel la teneur en oxyde de sodium $Na_2O$ est inférieure à 0,3% poids par rapport à la masse totale de l'adsorbant.

7. Adsorbant selon l'une des revendications précédentes, dans lequel la teneur totale en oxydes d'ions alcalins ou alcalino-terreux autres que l'oxyde de baryum BaO, l'oxyde de potassium $K_2O$, l'oxyde de strontium SrO et l'oxyde de sodium $Na_2O$ est inférieure à 1 % poids, bornes incluses, par rapport à la masse totale de l'adsorbant.

8. Adsorbant selon l'une des revendications précédentes, ledit adsorbant ayant un diamètre moyen en nombre compris entre 0,2 mm et 2 mm, bornes incluses.

9. Adsorbant selon l'une des revendications précédentes, dans lequel le diamètre moyen en nombre des cristaux de zéolithes X est inférieur ou égal à 1,5 $\mu$m bornes incluses.

10. Adsorbant selon l'une des revendications précédentes, dans lequel la fraction massique de zéolithe X est d'au moins 80% poids de zéolithe(s) X par rapport à la masse totale de l'adsorbant.

11. Procédé de préparation d'un adsorbant selon l'une quelconque des revendications précédentes, comprenant au moins les étapes de :

a) agglomération d'une poudre de cristaux de zéolithe X avec un liant, et mise en forme, puis séchage et calcination,
b) zéolithisation éventuelle du liant,
c) échange cationique de l'aggloméré, de manière simultanée, séquencée ou alternée par mise en contact avec des solutions contenant les ions baryum, les ions potassium ou les ions strontium seuls ou en mélange, en une ou plusieurs fois, puis lavage et séchage de l'aggloméré ainsi traité, et
d) activation de l'adsorbant zéolithique ainsi obtenu.

12. Procédé selon la revendication 11 **caractérisé en ce que** le liant mis en œuvre dans l'étape a) contient au moins 80 % en poids d'argile zéolithisable, une source de silice, et **en ce que** le procédé comprend une étape b) de zéolithisation dudit liant zéolithisable par action d'une solution basique alcaline, de préférence avec une solution de concentration comprise entre 0,5 et 5M, de préférence entre 0,5 M et 2 M et pendant une durée comprise entre quelques dizaines de minutes et quelques heures.

13. Utilisation d'un adsorbant selon l'une quelconque des revendications 1 à 10, dans les procédés de :

• séparation de coupes d'isomères aromatiques en C8, notamment des xylènes, et plus particulièrement de para-xylènes,
• séparation d'isomères de toluène substitué tels que nitrotoluène, diéthyltoluène, toluènediamine, et autres,
• séparation des crésols,
• séparation des alcools polyhydriques.

14. Procédé de récupération de para-xylène à partir de coupes d'isomères d'hydrocarbures aromatiques contenant 8 atomes de carbone, en phase liquide ou en phase gazeuse, par adsorption du para-xylène, comprenant les étapes successives suivantes :

a) une étape de mise en contact de la charge avec un lit d'adsorbant comprenant au moins un adsorbant zéolithique tel que défini selon l'une quelconque des revendications 1 à 10,
b) une étape de mise en contact du lit d'adsorbant avec un désorbant, de préférence choisi parmi le toluène et le para-diéthylbenzène,

de préférence, le procédé étant de type lit mobile simulé, plus préférentiellement à contre-courant simulé.

**Patentansprüche**

1. Zeolithisches Adsorptionsmittel, umfassend Zeolith-X-Kristalle und umfassend Barium, Kalium, Strontium und Natrium, wobei das molare Verhältnis $K_2O$ / $(K_2O + SrO + BaO + Na_2O)$ der Substanzen in Form von Oxiden zwischen 1,5 % und 8,0 %, Grenzwerte inbegriffen, liegt und das molare Verhältnis $SrO$ / $(K_2O + SrO + BaO + Na_2O)$ der Substanzen in Form von Oxiden zwischen 0,5 % und 8,0 %, Grenzwerte inbegriffen, liegt, wobei vorzugsweise die Zeolith-X-Kristalle ein Atomverhältnis Si/Al haben, das zwischen 1,00 und 1,50, Grenzwerte inbegriffen, liegt.

2. Adsorptionsmittel nach Anspruch 1, wobei das molare Verhältnis $K_2O$ / $(K_2O + SrO + BaO + Na_2O)$ der Substanzen in Form von Oxiden zwischen 2,0 % und 7,0 %, vorzugsweise zwischen 2,5 und 6,0 %, in sehr bevorzugter Weise zwischen 2,5 % und 4,0 %, Grenzwerte inbegriffen, liegt.

3. Adsorptionsmittel nach einem der Ansprüche 1 oder 2, wobei das molare Verhältnis $SrO$ / $(K_2O + SrO + BaO + Na_2O)$ der Substanzen in Form von Oxiden zwischen 0,5 % und 7,0 %, vorzugsweise zwischen 1,0 % und 6 %, in sehr bevorzugter Weise zwischen 1,0 und 4,0%, in noch bevorzugterer Weise zwischen 1,0 und 3,0 %, Grenzwerte inbegriffen, liegt.

4. Adsorptionsmittel nach einem der vorangehenden Ansprüche, wobei das molare Verhältnis $SrO$ / $K_2O$ der Substanzen in Form von Oxiden zwischen 0,3 und 2,0, vorzugsweise zwischen 0,35 und 1,5, in bevorzugter Weise zwischen 0,4 und 1,0, Grenzwerte inbegriffen, liegt.

5. Adsorptionsmittel nach einem der vorangehenden Ansprüche, umfassend ferner eine nicht-zeolithische Phase.

6. Adsorptionsmittel nach einem der vorangehenden Ansprüche, wobei der Gehalt an Natriumoxid $Na_2O$ unter 0,3 Gew.-% im Verhältnis zur Gesamtmasse des Adsorptionsmittels liegt.

7. Adsorptionsmittel nach einem der vorangehenden Ansprüche, wobei der Gehalt insgesamt an Oxiden alkalischer oder erdalkalischer Ionen, die andere als Bariumoxid BaO, Kaliumoxid $K_2O$, Strontiumoxid SrO und Natriumoxid $Na_2O$ sind, unter 1 Gew.-%, Grenzwerte inbegriffen, im Verhältnis zur Gesamtmasse des Adsorptionsmittels, liegt.

8. Adsorptionsmittel nach einem der vorangehenden Ansprüche, wobei das Adsorptionsmittel einen zahlenmittleren Durchmesser hat, der zwischen 0,2 mm und 2 mm, Grenzwerte inbegriffen, liegt.

9. Adsorptionsmittel nach einem der vorangehenden Ansprüche, wobei der zahlenmittlere Durchmesser der Zeolith-X-Kristalle kleiner oder gleich 1,5 $\mu$m, Grenzwerte inbegriffen, beträgt.

10. Adsorptionsmittel nach einem der vorangehenden Ansprüche, wobei der Massenanteil von Zeolith X mindestens 80 Gew.-% Zeolith(e) X im Verhältnis zur Gesamtmasse des Adsorptionsmittels beträgt.

11. Verfahren zur Herstellung eines Adsorptionsmittels nach einem der vorangehenden Ansprüche, umfassend mindestens die folgenden Schritte:

    a) Agglomerieren eines Zeolith-X-Kristallpulvers mit einem Bindemittel und Formgebung, dann Trocknung und Calcinierung,
    b) eventuelles Zeolithisieren des Bindemittels,
    c) Kationenaustausch des Agglomerats gleichzeitig, sequenziert oder alternierend durch Inkontaktversetzen mit Lösungen, die die Bariumionen, die Kaliumionen oder die Strontiumionen allein oder im Gemisch enthalten, in einem Mal oder mehrmals, dann Reinigung und Trocknung des derart behandelten Agglomerats, und
    d) Aktivieren des derart erhaltenen zeolithischen Adsorptionsmittels.

12. Verfahren nach Anspruch 11, **dadurch gekennzeichnet, dass** das in Schritt a) verarbeitete Bindemittel mindestens 80 Gew.-% zeolithisierbaren Ton, eine Siliciumoxidquelle enthält und dass das Verfahren einen Schritt b) des Zeolithisierens des zeolithisierbaren Bindemittels durch Wirken einer alkalischen basischen Lösung, vorzugsweise mit einer Lösung in einer Konzentration zwischen 0,5 und 5 M, vorzugsweise zwischen 0,5 M und 2 M und während einer Dauer umfasst, die zwischen einigen zehn Minuten und einigen Stunden liegt.

**13.** Verwendung eines Adsorptionsmittels nach einem der Ansprüche 1 bis 10 in folgenden Verfahren:

- Separieren von Schnitten aromatischer C8-Isomere, vor allem der Xylene, und insbesondere von para-Xylenen,
- Separieren von Isomeren substituierten Tuolens wie Nitrotoluen, Diethyltoluen, Toluendiamin und anderen,
- Separieren der Cresole,
- Separieren der mehrwertigen Alkohole.

**14.** Verfahren zur Rückgewinnung von para-Xylen aus Schnitten von Isomeren aromatischer Kohlenwasserstoffe mit 8 Kohlenstoffatomen in flüssiger Phase oder in gasförmiger Phase durch Adsorption des para-Xylens, umfassend die folgenden aufeinanderfolgenden Schritte:

a) einen Schritt des Inkontaktversetzens der Charge mit einem Adsorptionsmittelbett, umfassend mindestens ein zeolithisches Adsorptionsmittel nach einem der Ansprüche 1 bis 10,
b) einen Schritt des Inkontaktversetzes des Adsorptionsmittelbetts mit einem Desorptionsmittel, das vorzugsweise aus Toluen und para-Diethylbenzol ausgewählt ist,

wobei das Verfahren vorzugsweise vom Typ simuliertes bewegliches Bett, vorzugsweise mit simuliertem Gegenstrom, ist.

**Claims**

**1.** Zeolite adsorbent comprising zeolite X crystals and comprising barium, potassium, strontium and sodium, wherein the $K_2O$ / ($K_2O$ + SrO + BaO + $Na_2O$) molar ratio of the species in oxide form is comprised between 1.5% and 8.0%, limits included, and the SrO / ($K_2O$ + SrO + BaO + $Na_2O$) molar ratio of the species in oxide form is comprised between 0.5% and 8.0%, limits included, preferably the zeolite X crystals having an Si/Al atomic ratio comprised between 1.00 and 1.50 limits included.

**2.** Adsorbant according to claim 1, wherein the $K_2O$ / ($K_2O$ + SrO + BaO + $Na_2O$) molar ratio of the species in oxide form is comprised between 2.0% and 7.0%, preferably between 2.5% and 6.0%, very preferably between 2.5% and 4.0%, limits included.

**3.** Adsorbent according to one of claims 1 or 2, wherein the SrO / ($K_2O$ + SrO + BaO + $Na_2O$) molar ratio of the species in oxide form is comprised between 0.5% and 7.0%, preferably between 1.0% and 6%, very preferably between 1.0% and 4.0%, and even more preferably between 1.0% and 3.0%, limits included.

**4.** Adsorbent according to one of the preceding claims, wherein the SrO / $K_2O$ molar ratio of the species in oxide form is between 0.3 and 2.0, preferably between 0.35 and 1.5, more preferably between 0.4 and 1.0, limits included.

**5.** Adsorbent according to one of the preceding claims, also comprising a non-zeolite phase.

**6.** Adsorbent according to one of the preceding claims, wherein the content of sodium oxide $Na_2O$ is less than 0.3% by weight relative to the total mass of the adsorbent.

**7.** Adsorbent according to one of the preceding claims, wherein the total content of alkali metal or alkaline-earth metal ion oxides other than barium oxide BaO, potassium oxide $K_2O$, strontium oxide SrO and sodium oxide $Na_2O$ is less than 1% by weight, limits included, relative to the total mass of the adsorbent.

**8.** Adsorbent according to one of the preceding claims, said adsorbent having a number-average diameter comprised between 0.2 mm and 2 mm, limits included.

**9.** Adsorbent according to one of the preceding claims, wherein the number-average diameter of the zeolite X crystals is less than or equal to 1.5 $\mu$m, limits included.

**10.** Adsorbent according to one of the preceding claims, wherein the mass fraction of zeolite X is at least 80% by weight of zeolite(s) X relative to the total mass of the adsorbent.

**11.** Process for preparing an adsorbent according to any one of the preceding claims, comprising at least the steps of:

a) agglomeration of a powder of zeolite X crystals with a binder, and forming, then drying and calcining,
b) optional zeolitization of the binder,
c) simultaneous, sequential or alternating cation exchange of the agglomerate by putting in contact with solutions containing barium ions, potassium ions or strontium ions alone or as a mixture, one or more times, then washing and drying of the agglomerate thus treated, and
d) activation of the zeolite adsorbent thus obtained.

12. Process according to claim 11, **characterized in that** the binder used in step a) contains at least 80% by weight of zeolitizable clay, a source of silica, and **in that** the process comprises a step b) of zeolitization of said zeolitizable binder by the action of an alkaline basic solution, preferably with a solution having a concentration comprised between 0.5 and 5 M, preferably between 0.5 M and 2 M and for a period comprised between a few tens of minutes and a few hours.

13. Use of an adsorbent according to any one of claims 1 to 10, in processes for:

   • separating fractions of aromatic C8 isomers, in particular xylenes, and more particularly para-xylenes,
   • separating isomers of substituted toluene, such as nitrotoluene, diethyltoluene, toluenediamine, or the like,
   • separating cresols,
   • separating polyhydric alcohols.

14. Process for recovering *para*-xylene from fractions of aromatic hydrocarbon isomers containing 8 carbon atoms, in the liquid phase or in the gas phase, by adsorption of the *para*-xylene, comprising the following successive steps:

   a) a step of putting the feedstock in contact with a bed of adsorbent comprising at least one zeolite adsorbent as defined according to any one of claims 1 to 10,
   b) a step of putting the bed of adsorbent in contact with a desorbent, preferably chosen from toluene and *para*-diethylbenzene,

   preferably the process being of simulated moving bed type, more preferably in simulated counter-current mode.

# RÉFÉRENCES CITÉES DANS LA DESCRIPTION

*Cette liste de références citées par le demandeur vise uniquement à aider le lecteur et ne fait pas partie du document de brevet européen. Même si le plus grand soin a été accordé à sa conception, des erreurs ou des omissions ne peuvent être exclues et l'OEB décline toute responsabilité à cet égard.*

**Documents brevets cités dans la description**

- US 3558730 A
- US 3663638 A
- US 3960774 A
- US 2985589 A
- US 3626020 A
- US 3997620 A
- WO 2017005907 A
- FR 2789914

- US 7785563 B
- WO 2013106816 A
- US 5284992 A
- US 5629467 A
- US 4402832 A
- US 4498991 A
- FR 2999098

**Littérature non-brevet citée dans la description**

- « Principles of Adsorption and Adsorption Processes » (« Principes de l'Adsorption et des Procédés d'Adsorption »). John Wiley & Sons, 1984, 326, , 407
- **INAYAT.** *Angew. Chem. Int. Ed.,* 2012, vol. 51, 1962-1965
- **D. VERBOEKEND.** *Adv. Funct. Mater.,* 2012, vol. 22, 916-928

- **D.W. BRECK.** Zeolite Molecular Sieves. John Wiley and Sons, 1973, 314-315
- Principles of Adsorption and Adsorption Processes. John Wiley & Sons, 1984
- **MAZOTTI ; STORTI ; MORBIDELLI.** Robust Design of Countercurrent Adsorption Séparation Processes: 2. Multicomponent Systems. *AIChE Journal,* Novembre 1994, vol. 40 (11